# EUROPEAN PATENT APPLICATION

(11) **EP 2 851 066 A1**
(43) Date of publication of application: **25.03.2015**
(21) Application number: 13185631.2
(22) Date of filing: 23.09.2013
(51) Int. Cl.: A61K 9/107, A61K 9/00, A61M 5/30

(54) **Fixation of vaccine formulations on devices for epidermal immunisation by oily adjuvants**

(71) Applicant: LTS LOHMANN Therapie-Systeme AG, 56626 Andernach (DE)
(72) Inventor: Lell, Peter, 85368 Moosburg (DE); Winter, Gerhard, 82377 Penzberg (DE); Engert, Julia, 80993 München (DE); Etzl, Elsa, 79110 Freiburg (DE)
(74) Representative: Simandi, Claus

(57) **Abstract**

The present patent application relates to a pharmaceutical product and a device for the storage, handling, transport and administration of active ingredients, pharmaceutical formulations and vaccines. In addition, the present invention relates to a device and pharmaceutical product suitable for intra-dermal, intra-mucosal or epidermal administration like for example intra-dermal or epidermal immunisation. Further, the invention confers methods of attaching active ingredients, pharmaceutical formulations and vaccines to a surface by oily substances and the surfaces, pharmaceutical products and devices obtained by these methods. In particular, the present invention provides a device or pharmaceutical product for the attachment of vaccines to surfaces using oily substances that have at the same time an adjuvant effect.

## Description

### Field of the Invention

The present patent application relates to a pharmaceutical product and a device for the storage, handling, transport and administration of active ingredients, pharmaceutical formulations and vaccines. In addition, the present invention relates to a device and pharmaceutical product suitable for intra-dermal, intra-mucosal or epidermal administration like for example intra-dermal or epidermal immunisation. Further, the invention confers methods of attaching active ingredients, pharmaceutical formulations and vaccines to a surface by oily substances and the surfaces, pharmaceutical products and devices obtained by these methods. In particular, the present invention provides a device or pharmaceutical product for the attachment of vaccines to surfaces using oily substances that have at the same time an adjuvant effect.

All publications herein are incorporated by reference to the same extent as if each individual publication or patent application was specifically and individually indicated to be incorporated by reference. The following description includes information that may be useful in understanding the present invention. It is not an admission that any of the information provided herein is prior art or relevant to the presently claimed invention, or that any publication specifically or implicitly referenced is prior art.

In the last years novel methods for the administration of vaccine formulations have been developed, for example to avoid the use of needles and subcutaneous or intramuscular injections. These application methods have the drawback of undesired needle-stick injuries, unwanted spreading of infectious diseases by re-use of needles, and poor patient compliance due to needle phobia. More sophisticated vaccine delivery systems target the epidermis where a large number of immuno-competent cells, so-called Langerhans cells, are located. The administration via the skin can be accomplished by jet injection of liquids or by epidermal powder immunisation or by impregnated so called "microneedle arrays" where numerous very small needle like protrusions on a holder are used to puncture the uppermost layers of the skin and to reach the epidermis. These systems have the advantage that the administration is basically pain free and the amount of antigen required to elicit a sufficient immune response towards a vaccine relatively low when compared to the traditional administration via injection. Furthermore, when using solid or powder vaccines that have not to be dissolved resulting in a solution or suspension before use, the stability of such vaccines is increased compared to liquid vaccines or on the other hand the handling of such powder vaccines is strongly improved compared to systems that require re-dispersion. There is an on-going request to further develop the methods and techniques of epidermal powder immunisation and to improve the application devices. In general, the powder particles used for epidermal powder immunisation should display a certain size in the micrometre range (ideally between 10 - 100 µm, for example 20 - 80 µm, preferably 25 - 50 µm) in addition to sufficient density when it is intended to inject these particles by ballistic acceleration and bombardment into the skin.

A device for epidermal powder injection (EPI) has been described (e.g. see review by Kis et al.). In this device the powder particles are filled into a vaccine cassette comprised of a polyurethane thermoplastic elastomer washer and sealed at both ends with polycarbonate rupture membranes.

The cassette is attached to the device, and upon actuation, the valve of a helium microcylinder brakes, helium gas is released and the membranes of the vaccine cassette are ruptured. The device has the disadvantage that the particles are filled into the cassette in form of loose bulk powder and movement of the particles within the cassette is not constricted and therefore the distribution of the powder particles within the cassette can be irregular. This irregularity of particle distribution within the cassette can result in differences in the acceleration of the particles by the actuator; hence the velocity of the particles can be different as well as the impact and the resulting distribution depths within the skin.

Modifications of this device are described in Liu and Kendall, 2008, using a special converging-diverging supersonic nozzle (CDSN), or, as described in Liu et al. 2008, a system based on a contoured shock-tube (CST). In the modified device the vaccine is stored in a vaccine cassette. The membranes of the cassette are ruptured by the released helium shock wave and propelled down the tube. The particles are further transported by the gas flow and accelerated.

WO 2004/071558 A1, EP 1 599 242 B1, DE 10306715 A1 and AT 392913 E describe the development of a device for the injection of dust or powder formulations into a tissue; especially of a dust- or powdered drug or naked DNA using a pyrotechnically driven device powder injection. In this device the powder is either situated in a compartment which is on one side confined by a shiftable piston and on the other side by a membrane. The piston is accelerated towards the membrane which ruptures because of the pressure; hence the powder for injection is transported towards the opening of the device. In another embodiment the patent further describes the development of a device that allows for the application of small quantities of powder that can be attached to a membrane by adhesion forces. The described device allows even more different geometric forms of acceleration devices and besides that one can consider any form of modification on the geometry of such devices.

What these devices have in common, even with devices comprising micro needle arrays and similar type of devices is the need for an optimal way to attach the vaccine dose in its solid, potentially powdered form to the relevant surface of the device in a way that allows safe and stable storage, retains the dose at the surface of the device part even under mechanical stress as it is applied during manufacture, transport, storage and handling of such a device and releases the solid vaccine dose from this attachment situation to a full or at least fully sufficient extent when actuated for injection into the skin. In particular, the attachment force must be so strong that mechanical stress as e.g. during transport or during repeated falling of the device from a certain height must not result in relevant loss of the dose from the specific holder surface of the device. On the other hand the particular attachment force must be low enough that acceleration of the driver in the device can remove the full or at least the necessary amount of the dose from the device and release it into the target zone of the skin.

A further desired feature of the adhesion system is that it is composed of safe and on the other hand cheap and easy to handle materials that do not compromise or disturb the stability and or quality of the vaccine. The application of the adhesion material shall preferably by easy as for example with techniques like spraying on, dipping, wiping on etc. without the need of further compounds like solvents etc. of process steps like intermediate drying, curing etc.

Hence, there is still a strong need for novel devices and methods for transport, storage and administration of pharmaceutical formulations, in particular for novel possibilities of epidermal and intradermal administration of vaccines.

The applicant surprisingly found, that the attachment of active ingredients, like for example a pharmaceutical formulation or vaccine to a device or a pharmaceutical product by oily substances meets this need. In such a device or pharmaceutical product the active ingredient is attached by adhesive forces which are strong enough to allow safe and stable storage and which retain the dose on the surface of the device / pharmaceutical product even under mechanical stress during manufacture, transport, storage and handling of such a device / pharmaceutical product and which are on the other hand weak enough to releases the active ingredient from the device / pharmaceutical product to a full or at least fully sufficient extent during administration. The attachment force of the oily substance provokes that mechanical stress as during repeated falling of the device / pharmaceutical product from a certain height does not result in relevant loss of the dose from the surface of the device / pharmaceutical product. On the other hand the particular attachment force is low enough that for example by acceleration of a driver in the device full or at least the necessary amount of the dose of the active ingredient can be removed from the device and released into the target zone of administration.

A further advantage of attachment by oily substances is that such a device / pharmaceutical product is composed of safe, cheap and easy to handle material that does not compromise or disturb the stability and or quality of the active ingredient, the pharmaceutical formulation or vaccine.

The invention in general relates to a surface, wherein an active ingredient is attached to the surface by an oily substance.

Thus, in a first aspect, the present invention relates to a pharmaceutical product comprising at least one surface, at least one oily substance and at least one active ingredient, wherein the active ingredient is attached to the surface by the oily substance.

In a second aspect, the present invention relates to a device comprising at least one surface, at least one oily substance and at least one active ingredient, wherein the active ingredient is attached to the surface by the oily substance.

The surface, the pharmaceutical product and the device according to the invention allow the transport, handling, storage and / or administration of the active ingredient.

Preferably, the active ingredient is attached to the surface by adhesion forces.

In one embodiment of the invention the oily substance has an adjuvant effect.

In another embodiment of the invention the oily substance is an emulsion, like an emulsion comprising oil and water, for example a water-in-oil or an oil-in-water emulsion, preferably a water-in-oil emulsion. According to this embodiment the oily substance can be an emulsion comprising an oily phase and optionally an surfactant of an emulsion.

In another embodiment of the invention the oily substance comprises or consists of one or more oils selected from organic oils, mineral oils or synthetic oils, Miglyol, polyisobutylene (PIB), Freund's incomplete adjuvant, mannide mono-oleate, MF59®, silicone oil, paraffin oil, squalen, light mineral oil, vegetable oils, like for example coconut oil, palm oil, palm kernel oil, olive oil, soybean oil, canola oil, pumpkin seed oil, corn oil, sunflower oil, safflower oil, peanut oil, grape seed oil, sesame oil, argan oil, rice bran oil, avocado oil, caraway oil, castor oil, borage oil, cajeput oil, clove oil, cod oil, cottonseed oil, eucalyptus oil, evening primrose oil, fennel oil, lavender oil, olive oil, peanut oil, peppermint oil, rapeseed oil, tea tree oil, thyme oil. In a preferred embodiment of the invention the oily substance comprises or consists of MF59®.

Furthermore, the oily substance may comprise or consist of oily lipophilic substances or oily hydrophobic substances.

The oily substance can furthermore comprise further components or excipients like for example surfactants like lectin, Polysorbat, Span, Tween, antioxidants, chelators, fluidizers and others.

In a particular embodiment the oily substance is used in an amount of 0.1 - 50% of the total particle dose, preferable in an amount of 0,5 - 30%, more preferably in an amount of 1-25%, most preferably in an amount of 2 - 20%.

In another embodiment of the invention the active ingredient is part of a pharmaceutical formulation or a vaccine (vaccine formulation).

In another embodiment of the invention the active ingredient, the pharmaceutical formulation or the vaccine is in the form of a solid, a powder, a loose bulk powder, a fine granulate or pellet, a micropellet, a dust or in the form of microparticles or nanoparticles or microcrystals. The microparticles, nanoparticles or microcrystals can be of regular shape or irregularly shaped, e.g. like milled powders. In a preferred embodiment the microparticles, nanoparticles or microcrystals comprise a vaccine. The particles may have a particular size like 5 - 100 µm, for example 10 - 90 µm or 20 - 70 µm, preferably 25 - 50 µm in diameter.

In another embodiment of the invention the active ingredient is selected from the group of compounds comprising chemical compounds, extracts of natural origin like plant extracts or fractions of those extracts, biomolecules like proteins, peptides, antigenes, antibodies, steroids, hormones, nucleic acids, DNA, RNA, oligonucleotides, attenuated virus or bacteria, all types of vaccines, the named molecules themselves or parts thereof, like for example epitopes or paratopes, or being in a living organism, being part of a living organism, being part of a virus or being part of a part of a virus, being part of a DNA, RNA or other biotech molecule or of a part of such molecule. In another embodiment of the invention, two or more different active ingredients are used ("hybrid molecular mixtures"). The active ingredients of such hybrid molecular mixtures can be selected from the same class of compounds (e.g. both active ingredients are selected from the class of antigens, but both antigens are different) or from different classes of compounds (e.g. one active ingredient is selected from the class of antigens and another active ingredient is selected from the class of oligonucleotides).

In a preferred embodiment the active ingredient is in the form of a vaccine and comprise one or more antigens. In this case, the oily substance is used for example in an amount of 0.1- 50% of the total particle dose of the vaccine, preferable in an amount of 0,5 and 30%, more preferably in an amount of 1-25%, most preferably in an amount of 2-20%. In the case of a vaccine, the oily substance preferably is an oily adjuvant. An oily adjuvant is an oily substance that has an adjuvant effect, like e.g. MF59®. Vaccine particles can be composed of different materials and structures. They may for example be sugar based particles or polymer based particles. Preferably, vaccine particles are storage stable in the presence of the oily adjuvant. In addition, the oily adjuvant may prevent vaccine particles from moisture.

The another embodiment of the invention the surface is a smooth surface or a structured surface.

The another embodiment of the invention the surface is a solid support or a membrane.

The another embodiment of the invention the surface is a metal membrane, like an aluminium membrane or a titan membrane, a plastic material, a polymer, for example Polypropylene (PP), Polyethylene (PE), Polyoxymethylene (POM), a Polyether ether keton (PEEK) or Teflon.

Particles, consisting of or comprising the active ingredient, for example vaccine particles may be loaded onto the surface after the oily substance. In another particular embodiment the active ingredient, e.g. the vaccine and the oily substance are loaded onto the device and then the loading compartment is closed hermetically. The hermetically closed loading compartment can be opened before using the device to accelerate the active ingredient, e.g. the vaccine towards the target, e.g. the respective skin or tissue. The oily substance is accelerated towards the target in a way that it travels together with the active ingredient, e.g. the vaccine particles. Alternatively, the oily substance travels independently from the active ingredient, e.g. vaccine particles towards the target or remains on the surface while the active ingredient travels to the target.

In a preferred aspect, the invention relates to the delivery of the active ingredient, the pharmaceutical formulation or the vaccine to a target. Therefore, the particles are attached to an acceleration device by means of the oily substance or the surface comprising the active ingredients is attached to such acceleration device.

In another embodiment of the invention the device comprises an acceleration device, for example an acceleration surface or an acceleration membrane, a pyrotechnically driven powder injection device, a ballistic accelerator or a ballistic powder injection device. The active ingredient, the pharmaceutical formulation or the vaccine is attached to the surface of the acceleration device, the pyrotechnically driven powder injection device, the ballistic accelerator or the ballistic powder injection device by the oily substance. In a preferred embodiment the acceleration device acts via pyrotechnical acceleration.

The acceleration device can be used for ballistic delivery of the active ingredient, the pharmaceutical formulation or the vaccine into skin layers or into tissue layers (target tissue), for example into mucosa.

The surface may have all kinds of shape. The surface can be flat or curved, for example in a convex manner. In another example, the outer surface of the device can be flat and disk - shaped. Also more complex shapes are possible, e.g. party flat or partly curved.

For example, the particles can be attached to a flat or a curved surface on the acceleration device. If the surface has a more complex shape, the particles can be attached to a part of the surface.

The oily substance allows almost complete release of the particles from the surface of the device when accelerated by using the device for delivery. It allows fast enough release from the surface of the device to ensure penetration of the target tissue.

In order to produce the surface according to the invention, particles consisting of or comprising the active ingredient are loaded onto the surface. This could take place after the oily substance was brought onto the surface, which is one preferred embodiment. Alternatively, the particles could be brought into contact with the oily substance in a first step and in a second step the fully or partly by the oily substance covered particles are loaded onto the surface. In another particular embodiment the active ingredient and the oily substance are loaded onto the device simultaneously. The oily substance can be brought onto the surface for example by spraying on, dipping, wipping with or without the need of further compounds like solvents and / or additional process steps like intermediate drying or curing.

The surface could be part of a loading compartment. In a particular embodiment the loading compartment is closed, for example hermetically closed, after the active ingredient and the oily substance have been attached to the surface. The closed/hermetically closed loading compartment can be opened before using the device to accelerate the active ingredient towards the skin or other tissue.

The composition of the surface with respect to structure and material of the surface and the chosen oily substance allows to influence if the active ingredient travels together with the oily substance or if the active ingredient travel independently from the oily substance towards the target upon acceleration. The man of skill in the art can test this for example by using the methods described in the examples below. The oily adjuvant can for example be accelerated towards the target in a way that it travels together with the vaccine particles. On the other hand, the composition of the surface could render the oily adjuvant to travel independently from the vaccine particles towards the target.

The pharmaceutical product or the device are appropriate for use for epidermal administration, like for example intra-dermal or intra-mucosal administration or delivery. In case of an antigen or a vaccine the pharmaceutical product or the device are appropriate for use for immunisation, preferably for epidermal immunisation, like for example intra-dermal immunisation or intra-mucosal immunisation or epidermal powder immunisation (EPI).

In another aspect, the device is a ballistic drug delivery device comprising,
a) solid outer surface connected to an accelerator and
b) microparticles, nanoparticles or microcrystals containing or comprising an active ingredient and
c) an oily substance for attaching b) to a).

The invention further relates to the production and use of the surfaces, pharmaceutical products and devices.

In one aspect, the present invention relates to a method of attaching an active ingredient, a pharmaceutical formulation or a vaccine to a surface comprising the steps
a) enclosing particles of the active ingredient, the pharmaceutical formulation or the vaccine into a film of an oily substance thereby producing partly or full covered particles of the active ingredient, the pharmaceutical formulation or the vaccine and
b) bringing the partly or fully covered particles of the active ingredient, the pharmaceutical formulation or the vaccine into contact with the surface and
c) thereby attaching the active ingredient, the pharmaceutical formulation or the vaccine to the surface.

In another aspect, the present invention relates to a method of preparing a pharmaceutical product or a device according to the invention comprising the steps
a) enclosing particles of an active ingredient, a pharmaceutical formulation or a vaccine into a film of an oily substance thereby producing partly or full covered particles of the active ingredient, the pharmaceutical formulation or the vaccine and
b) bringing the partly or fully covered particles of the active ingredient, the pharmaceutical formulation or the vaccine into contact with the surface of the pharmaceutical product or the device and
c) thereby attaching the active ingredient, the pharmaceutical formulation or the vaccine formulation to the surface of the pharmaceutical product or the device.

In the context of the invention the oily substance has the function of an adhesion material. In another embodiment of the invention the oily substance has the function of an adhesive and an adjuvant. The oily substance can be either one having the function of adhesion or having both functions (adhesive and adjuvant) in one substance. The oily substance can be composed of more than one oily substance, one having the function of adhesion and another one having the function of adjuvant or any other possible combinations. The application of the oily substance is easy to perform. Therefore, the production of the pharmaceutical product or the device is also easy. They can for example be made by applying techniques like spraying on, dipping, wiping on. In a particular embodiment of the invention without the need of further compounds like solvents or process steps like for example intermediate drying or curing.

"Oily substance", as used herein means a substance that consists of an oil or a substance that comprises an oil. An oily substance may be an emulsion.

"Oil", as used herein means any substance that is an oil. An oil is an unctuous, combustible substance that is liquid, or liquefiable, on warming, and is soluble in ether but not in water. The oil may also be a wax. Oils may be solid at room temperature (RT) and soften or melt at temperatures slightly above RT. Oils may be animal, vegetable, or mineral in origin, or nonvolatile (fixed). Examples of oil are
borage oil extracted from the seeds of borage and used for the treatment of neurodermatitis and as a food supplement;
cajeput oil that is a volatile oil from the fresh leaves and twigs of cajeput;
canola oil obtained from rapeseed oil, specifically that prepared from rapeseed plants bred to be low in erucic acid;
castor oil which is a fixed oil obtained from the seed of Ricinus communis that is used as a bland topical emollient;
clove oil which is a volatile oil from cloves that is used externally in the treatment of colds and headache and as a dental antiseptic and analgesic;
corn oil which is a refined fixed oil obtained from the embryo of Zea mays that is used as a solvent and vehicle for various medicinal agents and as a vehicle for injections and that has also been promoted as a source of polyunsaturated fatty acids in special diets;
cottonseed oil which is a fixed oil from seeds of cultivated varieties of the cotton plant (Gossypium) that is used as a solvent and vehicle for drugs;
evening primrose oil which is produced from the ripe seeds of evening primrose(Oenothera biennis) and that is used in the treatment of mastalgia, premenstrual syndrome, and atopic eczema;
fennel oil which is a volatile oil distilled from fennel (the seeds of Foeniculum vulgare) and that is used for cough, bronchitis, and dyspepsia and as a pharmaceutical flavoring agent;
lavender oil which is a volatile oil distilled from the flowering tops of lavender or prepared synthetically and that is used for loss of appetite, dyspepsia, nervousness;
mineral oil which is a mixture of liquid hydrocarbons from petroleum; used as a lubricant laxative, drug vehicle, and skin emollient and cleanser;
light mineral oil which is of lesser density in comparison to mineral oil, is used similarly;
olive oil which is a fixed oil obtained from ripe fruit of Olea europaea and that is used as a setting retardant for dental cements, topical emollient, pharmaceutic necessity;
peanut oil which is the refined fixed oil from peanuts (Arachis hypogaea) and that is used as a solvent and vehicle for drugs;
rapeseed oil which is the oil expressed from the seeds of the rapeseed plant and that is used in the manufacture of soaps, margarines, and lubricants;
safflower oil which is an oily liquid extracted from the seeds of the safflower, Carthamus tinctorius, containing predominantly linoleic acid that is used as a pharmaceutic aid, a component of total parenteral nutrition solutions, and in the management of hypercholesterolemia;
silicone oil which comprises any of various long-chain fluid silicone polymers, some of which are injected into the vitreous to serve as a vitreous substitute during or after vitreoretinal surgery;
tea tree oil which is an essential oil from the leaves and branch tips of tea tree, having bacteriostatic and weak antiviral and antimycotic properties, used topically for skin infections and used internally and externally;
thyme oil which is extracted from fresh, flowering thyme that is used as an antitussive and expectorant.

The term "oil" may comprise all lipophilic substances of ther chemical nature; it also comprises expressed oil, fatty oil, fixed oil or nonvolatile oil, i.e., one that does not evaporate on warming. Such oils consist of a mixture of fatty acids and their esters, and are classified as solid, semisolid, and liquid, or as drying, semidrying, and nondrying as a function of their tendency to solidify on exposure to air.

The term "mineral oil" as used herein comprises any oil of various light hydrocarbon oils, especially a distillate of petroleum, a refined distillate of petroleum, a mixture of liquid hydrocarbons obtained from petroleum. Commonly used synonyms for mineral oil are heavy liquid petrolatum, liquid paraffin, liquid petroleum. Preferably used are mineral oils that are suitable for medicinal use for example as a laxative or stool softener. Mineral oil is available in both light grade (light liquid petrolatum) and heavier grades (liquid or heavy liquid petrolatum). Light mineral oil is used chiefly as a vehicle for drugs, but it may also be used as a cathartic and skin emollient and cleansing agent. Heavy mineral oil is used as a cathartic, solvent, and oleaginous vehicle.

MF59@ is a vaccines' adjuvant that was developed in the 1990s by researchers at Ciba-Geigy and is now proprietary to Novartis. MF59@ is the first oil-in-water adjuvant to be commercialized in combination with a seasonal influenza vaccine (Fluad®). MF59@ increases the immune response compared to non-adjuvanted vaccines. It has been tested extensively with more than 12 years of clinical experience and more than 45 million doses of adjuvanted vaccines distributed, MF59@ has an established safety profile and has been shown to be well tolerated in children, adults and the elderly. MF59@ is an immunologic adjuvant that uses squalene. It is added to influenza vaccines to help stimulate the human body's immune response through production of CD4 memory cells. In a preferred embodiment of the invention, MF59® is used as adjuvant. In another preferred embodiment of the invention MF59® is used as oily substance. In another preferred embodiment of the invention MF59@ is used as oily substance and adjuvant.

An "adjuvant", as used herein, is a pharmacological or immunological agent that modifies the effect of the active ingredient. It may be an inorganic or organic chemical, macromolecule or whole cells of certain killed bacteria. An adjuvant enhances the immune response to given antigen. Adjuvants are often included in vaccines to enhance the recipient's immune response to a supplied antigen, while keeping the injected foreign material to a minimum. Their purpose is to excite the immune system to a greater feedback than the vaccine itself, without the adjuvant. Firstly, there is greater "mobilization" of the immune system to the vaccine without which the organism had greater problems; adding adjuvant represents an opportunity to add the minimum amount of antigen to the body and the way to replace several repeated vaccinations by only one.

In a particular embodiment of the invention adjuvant means substances or active ingredients that may increase the efficacy or potency of other ingredients when given at the same time. Many different adjuvants are known in the literature and a certain number of them are in clinical and commercial use. Adjuvants ensure the proper strength of the immune response and without them often vaccination would be highly inefficient, i.e. would require immense doses or repeated vaccination. The adjuvants shall activate the immune system sufficiently to induce long-lasting and protective immune responses.

An "adjuvant effect", as used herein, means that the oily substance or the oil, respectively, functions as an adjuvant and therefore increase the efficacy or potency of the active ingredient.

An "emulsion" is defined as a dispersion of a liquid called the dispersed phase in a second liquid called the continuous phase with which the first one is not miscible. In vaccine formulations, these phases are on the one hand hydrophobic phase like for example water, PEG or other non-oil-miscible phase and on the other hand the oil. In order to stabilise the emulsions, surfactants can be added. A surfactant is a compound containing a polar group which is hydrophilic and a non polar group which is hydrophobic and often composed of a fatty chain. Surfactants can be defined by their hydrophilic / lipophilic Balance (HLB) value which gives an information on their relative affinity for the both phases. According to the HLB value of the surfactant, different kind of emulsions can be achieved. Those having a low HLB value have a high affinity for oily phases and render W/0 emulsions. In this case, the hydrophilic phase is made of droplets dispersed into the continuous oily phase. Those with a high HLB value have a high affinity for the aqueous phase and render 0/W emulsions, where the continuous phase is water and the dispersed phase is oil. At last, with certain specific surfactant systems, when the HLB value is intermediate, W/O/W emulsions can be achieved. In this case, the continuous phase is aqueous and the dispersed phase is oil. But inside the oil droplets, an entrapped aqueous phase is found. The viscosity of the emulsion is closely linked to the surfactant structure and its HLB value.

The particle size is also an important parameter influenced by an equation of the surfactant system and the emulsification process. It is generally recognised that emulsions having a small particle size and a homogeneous distribution are more stable. The methods to produce and optimize emulsions are known to the man of skill in the art.

The term "emulsion", as used herein, comprise water in oil (W/O) emulsions that induce a strong and long term immune response. Those based on mineral oils are known to be very efficient but can sometimes induce local reactions with reactive antigens. Non mineral oils are well tolerated but less efficient with poor immunogens. Multiphasic (W/O/W) emulsions can induce short and long term immune responses with various antigens and oil in water (O/W) emulsions are well tolerated and induce a short term immune response. The oily substance(s) and the surfactant(s) and other excipient that form an emulsion with water, or in other words: the emulsion without water.

An example for such an adjuvant is Montanide ISA 720 which is a mineral oil based emulsion that has been used as adjuvant for vaccination. Other type of adjuvants such as Montanide ISA 25D or 206D rendering, respectively O/W and W/O/W emulsions. MF59@ is another example for an oil-in- water-emulsion, that comprises squalen, surface-active polysorbat (Tween 80) and sorbitantrioleat (Span 85). After injection MF59@ is fast discharged to the lymph system, where it accelerates the uptake of vaccinating antigens to the immune system. MF59® is currently used in vaccine formulations Optaflu®, Fluad®, Focetria® and Celtura®.

"Vaccines" or "vaccine formulation" are composed of different ingredients, typically the antigen itself, being in a living organism, part of a living organism, virus or part of viruses, DNA, RNA or other biotech molecules or parts of such molecules, hybrid molecular mixtures of many of such molecules, viruses, parts of viruses, bacteria, etc.. Besides that vaccines may contain further excipients for example to provide their stability, allow their manufacturability, their microbiologically safe quality. Furthermore, a typical compound of a vaccine, either formulated with the vaccine or, often, although inconvenient in handling and difficult to standardize, provided separately to the vaccine and admixed to it before use is the adjuvant.

This approach underlying the present invention can be applied easily by a man skilled in the art to known and new adjuvants. The man of skill in the art will also easily find the right adjuvant for any particular vaccine and can combine it with the vaccine in the right form to ensure storage stability as well as efficacy, safety and pharmaceutical quality.

The present invention allows to use low doses of active ingredients. Preferably, also the overall dose of the adjuvants should be kept as low as possible and of course as high as necessary, a compromised that is often hard to find due to certain incompatibilities of certain adjuvants with liquid vaccines, instability of the adjuvants themselves. The man of skill in the art can determine the right dose of adjuvants, oily substance and active ingredient.

The "target" is the area of administration for which the device or the pharmaceutical product is suitable. Targets are preferably tissue of humans or animals, including but not limited to wound tissue, gums, nails, teeth, bone, tendons, cartilage, epidermis or mucosa. In a preferred embodiment the target is a particular layer of the epidermis. The epidermis is composed of 4 or 5 layers depending on the region of skin being considered. Those layers in descending order are: cornified layer (stratum corneum), clear/translucent layer (stratum lucidum, only in palms and soles), granular layer (stratum granulosum), spinous layer (stratum spinosum), basal/germinal layer (stratum basale/germinativum). The term Malpighian layer (stratum malpighi) is usually defined as both the stratum basale and stratum spinosum.

The epidermis is separated from the dermis, its underlying tissue, by a basement membrane. In another preferred embodiment the target is mucosa. The mucous membranes are linings of mostly endodermal origin, covered in epithelium, which are involved in absorption and secretion. They line cavities that are exposed to the external environment and internal organs. They are at several places contiguous with skin: at the nostrils, the lips of the mouth, the eyelids, the ears, the genital area, and the anus. Examples of potential target mucosa are buccal mucosa, nasal mucosa, oral mucosa, penile mucosa.

A "support" within the meaning of the invention is preferably a solid support. The solid support or matrix is typically glass or a polymer, the most commonly used polymers being cellulose, polyacrylamide, nylon, polystyrene, polyvinyl chloride or polypropylene. In all the embodiments, the term "support" covers embodiments such as a filter, a membrane, a bead, a silica wafer, glass, metal, ceramics, plastics, a chip, or a matrix. The solid supports may be in the form of tubes, beads, discs, silicon chips, microplates, polyvinylidene difluoride (PVDF) membrane, nitrocellulose membrane, nylon membrane, other porous membrane, non-porous membrane (eg. plastic, polymer, perspex, silicon, amongst others), or any other surface suitable for the administration of active ingredients.

A "formulation", within the meaning of the invention comprises pharmaceutical formulations and vaccines (vaccine formulation). The invention pertains to processes for the manufacture of medicinal drugs that are characterized by the feature that at least one active ingredient is brought into a suitable form of agent for administration together with a pharmaceutically suitable and physiologically tolerated vehicle and, optionally, further suitable active substances, additives, or ancillary substances. Suitable galenic forms of formulations are, for example, granulated materials, powders, (micro)capsules, suspensions, emulsions, microparticles, pellets, crystals, minirods, any solid microstructures as well as preparations with a protracted release of the active substance, whereby use is made in their preparation of conventional ancillary substances, such as vehicle substances, agents that lead to the disintegration of the preparation, binders, coating agents, swelling agents, slippage promoting agents or lubricants, taste improving agents, sweeteners, and solubilizers.

The pharmaceutical formulation or vaccine is preferably manufactured and administered in dosage units, whereby each unit contains, as the active ingredient, a defined dose. This dose can amount to 0,1 to 5 mg and preferably 0,2 to 4 mg or from 0.5 to 3 mg and preferably 1 to 2 mg.

Daily doses of 0,1 to 5 mg and preferably 0,2 to 4 mg or from 0.5 to 3 mg and preferably 1 to 2 mg of active ingredient, are indicated for the treatment of an adult patient weighing 50 to 100 kg, e.g. 70 kg. However, higher or lower daily doses can also be applied under certain circumstances. The administration of the daily dose can take place via an administration on one single occasion in the form of an individual dosage unit or several smaller dosage units, or via the multiple administration of subdivided doses at defined intervals or multiple administration of the full dose.

The present invention provide a solution to the demands articulated above in a way that a solid, preferentially powdered vaccine is attached to a surface of an acceleration device by means of a material that provides in parallel direct adjuvant activity.

More particularly, the approach comprises the attachment of vaccine powder particles to a surface or membrane with at least one compound, wherein the compound has adhesive properties and at the same time shows an adjuvant effect. The compounds according to the invention are oily substances that are functioning as adjuvants and can be applied between surface of the device and the solid (powdered) vaccine. Oily substances of the selected kind provide in parallel the features of been applicable by simple and cheap and reliable technologies and being as well safe and effective adjuvants.

Surprisingly, it was found that the compounds according to the invention show adhesive properties and attach the powder particles to different surfaces efficiently so that particles remain practically completely on the different surfaces made from different materials after different strong mechanical stresses, relevant to transport, manufacture, storage and handling situations.

The compounds have revealed unexpected adhesive efficiencies for attaching particles to different surfaces.

In addition to adhesive properties, the compounds also ensure nearly complete detachment of the particles from the different surfaces upon acceleration by the acceleration apparatus. The compounds are therefore well suited for attaching particles to different surfaces but at the same time also allow for detachment of the particles upon acceleration.

Surprisingly, only a small amount of the compounds was necessary for particle attachment. Particles were enclosed into a thin compound film, hence resulting in a partly or, if the amount of the compound was increased, full coverage of the surface of the vaccine particles. In some embodiments of the invention it can be achieved that the vaccine particles are protected from air, water / humidity, potentially also from light and other environmental factors.

Furthermore by combining oily adjuvants with solid vaccines on a device according to the invention a reduction of the adjuvant amount necessary for sufficient immunostimulatory effect can be achieved. Co-localisation of the oily adjuvant film to the particulate vaccine will ensure the effect of the adjuvant at the specific place where it is most effective. By that, neither adjuvant nor vaccine is lost or used inefficiently.

In comparison with the state of the art, the means of the inventions have the advantage of adhesion, full release at acceleration, the adjuvant with co-localisation, use of low dose, high stability, cost effectiveness and easy to be prepared.

The following figures and examples are intended to illustrate the invention without limiting the scope as a result.
Figure 1: Schematic illustration of the experimental set-up using a jolting volumeter, simulating the mechanical force acting on the device during shipping, storage and handling.
Figure 2: Remaining mass of particles and adhesive on aluminium membranes using the oily components of MF59®, silicon oil and paraffin oil as adhesives after 10, 20, 50, 100 and 500 beats of the jolting volumeter.
Figure 3: Schematic illustration of the custom made drop apparatus.
Figure 4: Adherence strength of glass particles on aluminium membranes (black bars) and PE-membranes (grey bars) using different adhesives.
Figure 5: Adherence strength of glass particles on perfectly smooth foils or structured surfaces using the oily components of MF59® as adhesive.
Figure 6: Schematic set-up of the ballistic accelerator (created by Dr. Lell, Pyroglobe GmbH).
Figure 7: (A) Photograph taken by a high speed PCO camera depicting two clouds of glass particles detached from the aluminium membrane and accelerated to high velocities. (B)

Light micrograph of the surface of the aluminium membrane after the experiment, showing that the glass particles were completely detached from the membrane surface.

Figure 8: Photographs taken by a high speed PCO camera depicting clouds of glass particles detached from the aluminium membrane attached with different adhesives.

### Example 1: Adherence of glass particles on aluminium membranes using oily adjuvants

The ability of oily components of adjuvants based on oil-in-water emulsions (e.g. MF59®) to act as an adhesive between particles and membrane surfaces was explored for the use in a ballistic powder injection device. The general concept underlying these experiments is that only the oil and the surfactant from those adjuvant emulsions were taken. Using this set up a high effect was expected due to the fact, that the compounds are finely dispersed 1.) by acceleration, 2.) by the surface of the microparticles and 3.) being in the pores of the microparticles.

The adhesive has to fulfil two requirements, firstly to attach the vaccine particles to the surface membrane of the device, second to be released together with the vaccine particles upon actuation of the device and third to elicit an appropriate immune response together with the antigen.

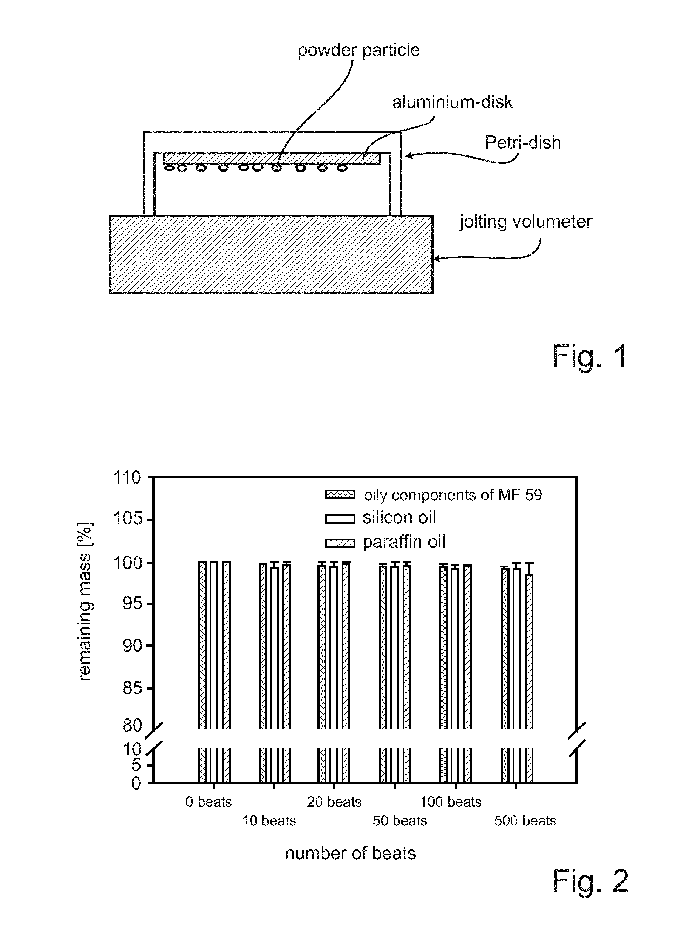

The oily components of MF 59, silicon oil and paraffin oil were explored as adhesives at the interface between aluminium membranes and glass particles. For the preparation of the oily components of the adjuvant MF 59 1500 µl squalene, 150 µl polysorbate 80 and 150 µl span 80 were mixed by vortexing in an Eppendorf tube. Glass particles were chosen as model particles for vaccine particles because of their well characterised and reproducible physical properties. Three aluminium membranes having a surface area of 6.28 cm² were covered with adhesive and dipped in a bed of glass particles having a particle size ranging from 20 µm to 40 µm and particle density of 2.5 g/cm³, so that the particles were fixed onto the membrane surface. The excess particles were tapped off. The mass of adhesive and particles was determined gravimetrically. The ratio between the mass of adhesive and the mass of particles was approximately 1:10. The appearance of the membrane's surface covered with the adhesive and the particles was visualized by optical microscopy. The mechanical force acting on the device during shipping, storage and handling was simulated using a jolting volumeter (figure 1). The membranes were fixed on the inner surface of a petri-dish according to instruction of manufacturer and were attached headfirst to a jolting volumeter with the particle covered side turned downwards. The border of the petri-dish served as a spacer to the surface of the jolting volumeter.

The mass of particles and adhesive remaining on the membranes after 10, 20, 50, 100 and 500 beats performed by the jolting volumeter was determined gravimetrically. After 500 beats 99.2% of the mass of glass particles was recovered using the oily components of MF 59 and silicon oil as adhesive, and 98.6% of the mass of glass particles was recovered using paraffin oil (figure 2), thus confirming the suitability of the adhesives to attach the particles onto the surface of aluminium membranes.

### Example 2: Adherence of glass particles on different membrane surfaces using oily adjuvants

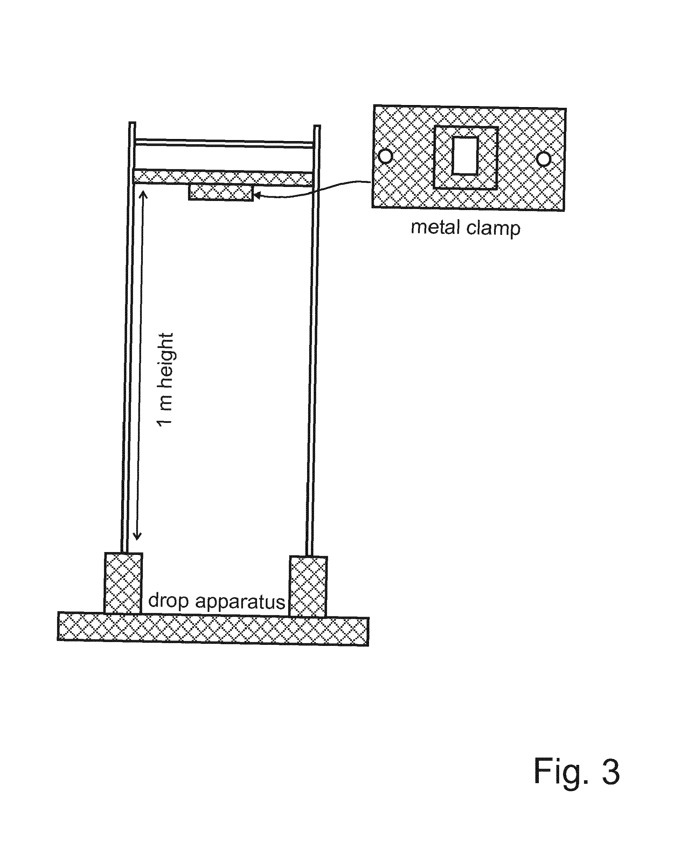

In order to increase the stress simulated by the jolting volumeter as explored in example 1, a custom made drop apparatus was used (figure 3). Therefore a piece of membrane coated with adhesive and particles as described in example 1 was fixed in a metal clam and dropped from one meter height. By the impact onto two distance pieces the metal clam was stopped abruptly from free-falling by a beat. The experiment was repeated 10 times with each three replicates. Thereby particles with a weak adherence were detached from the membrane surface. The remained particle and adhesive mass on the membrane was determined gravimetrically. Different materials suitable for the use as membrane surfaces in a ballistic powder injection device were explored. The membranes were covered with the mixture of the oily components of MF 59 and coated with glass particles as described in example 1.

**Table 1: Adherence strength of glass particles on different membrane materials using the oily components of MF59® as adhesive.**

| Membrane material | Remaining mass [ % ] |
|---|---|
| pp | 85 |
| PE | 86 |
| POM | 87 |
| PEEK | 84 |
| Al | 98 |

After 10 drops 97.9% ± 1.23 of the mass of particles and adhesive were recovered using Aluminium as membrane surface (table 1). Also the use of other plastic materials like polypropylene (PP), polyethylene (PE) polyoxymethylene (POM), polyether ether keton (PEEK) confirmed the suitability of the oily components of MF 59 as adhesive.

### Example 3: Adherence of glass particles on membrane surfaces using different adhesives

The oily components of MF59, paraffin oil, silicon oil, miglyol, castor oil, squalene, polyethyleneglycol 400, polyisobutylene and Freund's incomplete adjuvant, composed of 85% paraffin oil and 15% mannide monooleate, were investigated for the use as adhesive agent between glass particles and membranes. Two different membrane surface materials, aluminium and polyethylene membranes were covered with adhesive. The membranes were prepared according to example 1. The mechanical force acting on the device during shipping, storage and handling was simulated by dropping the membrane ten times from one meter height using a custom made drop apparatus as described in example 2.

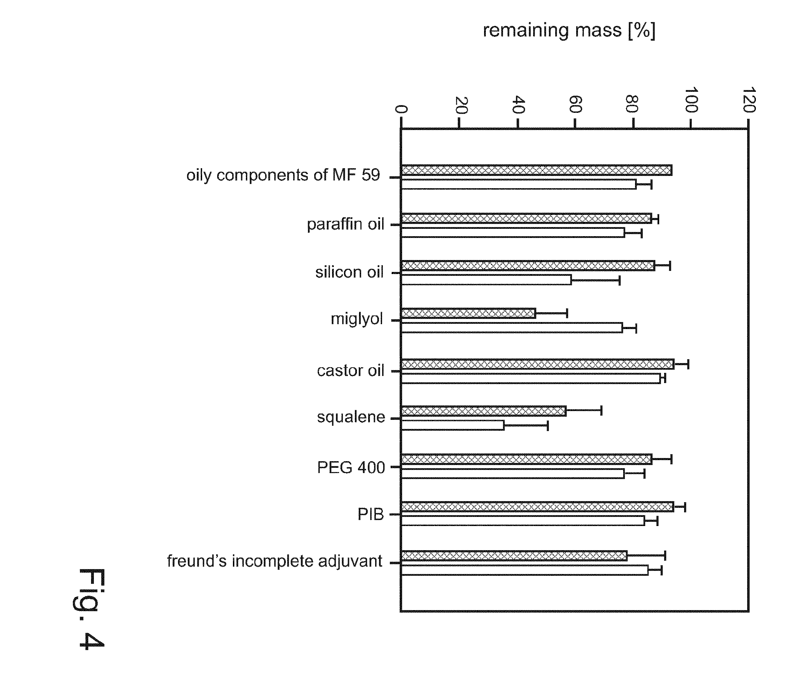

After 10 drops from one meter height (using a custom made drop-apparatus) the majority of the mass of particles and adhesive remained on the surface of aluminium membranes using the oily components of MF59, paraffin oil, silicon oil, castor oil, polyethylene glycol 400, polyisobutylene or Freund's incompletes adjuvant as adhesive is displayed in figure 4. The adherence to PE surfaces was comparable, in some case slightly higher or lower compared to aluminium surfaces.

### Example 4: Adherence strength of oily adjuvants on membranes with different surface structures

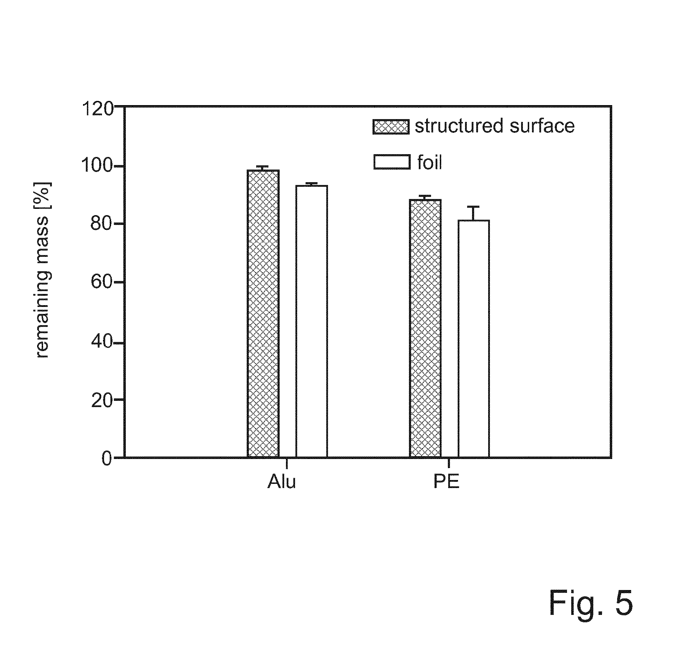

The adherence strength of glass particles on membranes is not only dependent on the type of adhesive and the membrane material but also on the roughness of the surface. The adherence strength of glass particles using the oily components of MF59 as adhesive on smooth surfaces was compared with the adherence strength on structured membrane surfaces (figure 5). Aluminium and polyethylene membranes were prepared as described in example 1 and the adherence strength of the particles was challenged by dropping the membrane ten times from one meter height using a custom made drop apparatus as described in example 2.

Regarding the adherence strength on aluminium and polyethylene membranes 98% and 88% of the glass particles, respectively, were retained using structured surface membranes, whereas 93% and 81% of the particles remained on perfectly smooth foils. The structure of the surface has a measurable effect but also on perfectly smooth surfaces, the retention of the material was very high.

### Comparative example A: Adhesion of glass particles on the surface of membranes by electrostatic interactions

Three different membrane materials (Teflon, aluminium and polyethylene) were dipped in a bed of glass particles having a particle size ranging from 20 µm to 40 µm and particle density of 2.5 g/cm³ in order to fix the glass particles onto the membrane surface without the use of an adhesive but by electrostatic interactions. The mass of glass particles was determined gravimetrically.

Likewise the three different membrane materials were covered the oily components of MF 59. The amount of adhesive was determined gravimetrically. The membranes covered with adhesive were dipped in a bed of glass particles. The excess glass particles were tapped off. The mass of glass particles was determined gravimetrically.

Without the application of an adhesive only 5.73 mg glass particles were attached to Teflon membranes, 0.27 mg to aluminium membranes and 2.17 mg to polyethylene membranes by mainly electrostatic interactions. However, 18.23 mg, 12.53 mg and 15.00 mg glass particles were attached to Teflon membranes, aluminium membranes and polyethylene membranes, respectively, using the oily components of MF 59 (table 2). This proves the ability of the oily components of MF 59 to attach a substantial mass of glass particles on different membranes made of several materials.

**Table 2: Adhesion of glass particles on the surface of polyethylene membranes by the use of adhesives or electrostatic interactions**

| Type of membrane | Remaining particle mass without the use of adhesive [mg] | Remaining particle mass using the oily components of MF 59 as adhesive [mg] |
|---|---|---|
| Teflon | 5.73 ± 1.42 | 18.23 ± 2.64 |
| aluminium | 0.27 ± 0.06 | 12.53 ± 3.16 |
| polyethylene | 2.17 ± 0.81 | 15.00 ± 6.10 |

### Example 5: Adherence of particles on membranes using oily adjuvants at different ambient temperature conditions

The oily components of MF59 was investigated for the use as adhesive agent between glass particles and membranes at different ambient temperature conditions. Two different membrane surface materials, aluminium and polyethylene membranes were covered with adhesive. The membranes were prepared according to example 1. The mechanical force acting on the device during shipping, storage and handling was simulated by dropping the membrane ten times from one meter height using a custom made drop apparatus as described in example 2. The samples as well as the custom made drop apparatus were tempered for two hours prior to the experiment to 2-8°C, 25°C or 40°C.

After 10 drops from one meter height more than 82.5% of the mass of particles and adhesive remained on the surface of aluminium and polyethylene membranes using the oily components of MF59 as adhesive, independently of the ambient temperature (table 3).

**Table 3: Adherence strength of glass particles on aluminium and polyethylene (PE) membranes using the oily components of MF59® as adhesive at 2-8°C, 25°C and 40°C.**

| Type of particle, storage temperature | Remaining mass [ % ] |
|---|---|
| MF59/Al, storage at 2-8°C | 92 |
| MF59/PE , storage at 2-8°C | 88 |
| MF59/Al , storage at 25°C | 92 |
| MF59/PE, storage at 25°C | 89 |
| MF59/Al, storage at 40°C | 86 |
| MF59/PE, storage at 40°C | 81 |

### Example 6: Adherence of particles on the surface of membranes using oily adjuvants after 12 weeks of storage at different ambient temperature conditions

The oily components of MF59 was investigated for the use as adhesive agent between glass particles and membranes after 12 weeks of storage at different ambient temperature conditions. Two different membrane surface materials, aluminium and polyethylene membranes were covered with adhesive. The membranes were prepared according to example 1. The mechanical force acting on the device during shipping, storage and handling was simulated by dropping the membrane ten times from one meter height using a custom made drop apparatus as described in example 2. The samples were re-tempered to room temperature prior to the experiment.

By using the oily components of MF59 as adhesive more than 60% of particles and adhesive were recovered after ten drops form one meter height after 12 weeks of storage at 25°C and 40°C (table 4).

**Table 4: Adherence strength of glass particles on aluminium and polyethylene (PE) membranes using the oily components of MF59® as adhesive after 12 weeks of storage at 25°C and 40°C.**

| Type of particle, storage temperature | Remaining mass [ % ] |
|---|---|
| MF59/Al , storage at 25°C | 60 |
| MF59/PE, storage at 25°C | 70 |
| MF59/Al, storage at 40°C | 78 |
| MF/PE, storage at 40°C | 82 |

### Example 7: Ability of the adhesive to assure the adherence of particles on the membrane surface during transport and storage, but release of the particles upon acceleration using a ballistic accelerator

Aluminium membranes were prepared as described in example 1. Aluminium membranes were covered with the oily components of MF 59 (as described in example 1) and glass particles of a size fraction of 20 µm to 40 µm were attached.

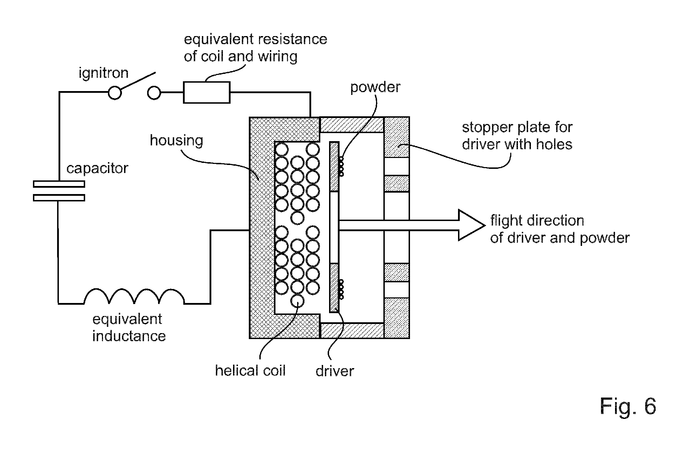

A ballistic accelerator was used to simulate the ballistic injection process. The basic working principle of the ballistic accelerator is the generation of a repulsive force by a magnetic field which induces a turbulent electrical current in a metal carrier, e.g. an aluminium membrane, which is thereby accelerated to high velocities. The aluminium membrane acts as carrier surface for the particles and is stopped by a stopper plate, while the particles attached on the membrane are further accelerated (figure 6).

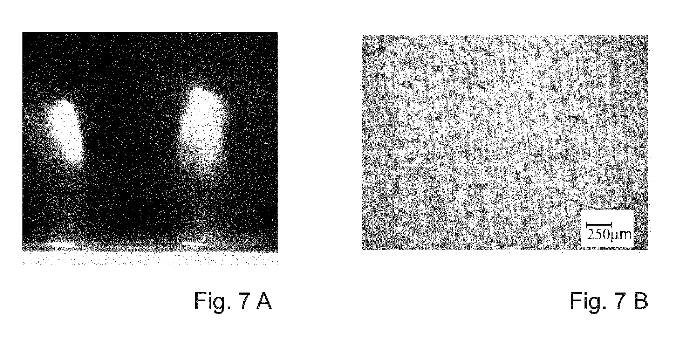

Upon acceleration using the ballistic accelerator glass particles attached with the oily components of MF 59 were completely detached from the aluminium membranes gaining high velocity above 100 m/s (figure 7 A and B). In figure 7B no glass particles are visible anymore on the aluminium membrane surface. This proves that the mechanical strength at the interface between glass particles and aluminium membranes using the oily components of MF 59 as adhesive is strong enough to avoid powder loss during storage and shipping; however the particles can be detached upon acceleration using a ballistic accelerator.

### Comparative Example B

The release of glass particles attached on aluminium membranes using the oily components of MF 59, polyisobutylene and superglue as adhesive upon acceleration using a ballistic acceleration (as described in example 7) was investigated. Glass particles were attached to aluminium membranes as described in example 1.

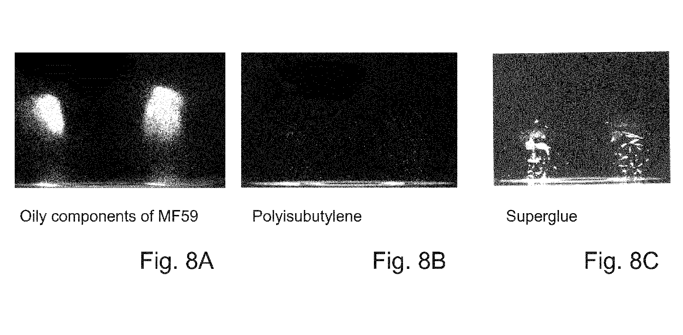

Particles attached with the oily components of MF 59 on aluminium membranes were completely detached upon acceleration using the ballistic accelerator (figure 8 A). Particles attached with polyisobutylene on aluminium membranes were not detached upon acceleration and remained almost completely on the membrane surface (figure 8 B). Particles attached with superglue were partially detached in form of solid pieces which consisted of aggregates of glue and particles (figure 8 C).

### Example 8: Entrainment of adhesive together with particles during detachment from the membrane and acceleration

A mixture of the oily components of MF 59 was prepared as described in example 1 and mixed with 1% Sudan red. Glass particles of a size fraction of 20 µm to 40 µm were attached onto aluminium membranes using the Sudan red died mixture of the oily components of MF 59 as described before. Particles were detached upon acceleration using the ballistic accelerator and caught on the surface of a second membrane. A light-micrograph of particles detached from the membrane surface during acceleration and caught on a second membrane. The glass particles were covered with Sudan red died adhesive, showing that a substantial amount of adhesive was entrained together with the particles during detachment from the membrane and acceleration. Therefore the adhesive can be delivered together with the particles into the target using a ballistic injection device. The distribution of the particles on the surface is equal, independently of the presence of the adhesive. That means, that the particles travel independently from each other as single particles but in case they are attached with an adhesive they take a certain amount of adhesive with them.

## Claims

1. Pharmaceutical product comprising at least one surface, at least one oily substance and at least one active ingredient, wherein the active ingredient is attached to the surface by the oily substance.

2. Device comprising at least one surface, at least one oily substance and at least one active ingredient, wherein the active ingredient is attached to the surface by the oily substance.

3. Pharmaceutical product or device according to claim 1 or 2, wherein the active ingredient is attached to the surface by adhesion forces.

4. Pharmaceutical product or device according to one of the previous claims, wherein the oily substance has an adjuvant effect.

5. Pharmaceutical product or device according to one of the previous claims, wherein the oily substance is an emulsion.

6. Pharmaceutical product or device according to one of the previous claims, wherein the oily substance comprises or consists of one or more oils selected from organic oils, mineral oils or synthetic oils, Miglyol, polyisobutylene (PIB), Freund's incomplete adjuvant, mannide mono-oleate, MF59®, silicone oil, paraffin oil, squalen, light mineral oil, vegetable oils, like for example coconut oil, palm oil, palm kernel oil, olive oil, soybean oil, canola oil, pumpkin seed oil, corn oil, sunflower oil, safflower oil, peanut oil, grape seed oil, sesame oil, argan oil, rice bran oil, avocado oil, caraway oil, castor oil, borage oil, clove oil, cod oil, cottonseed oil, evening primrose oil, fennel oil, lavender oil, olive oil, peanut oil, rapeseed oil, tea tree oil, thyme oil.

7. Pharmaceutical product or device according to one of the previous claims, wherein the active ingredient is part of a pharmaceutical formulation or a vaccine.

8. Pharmaceutical product or device according to one of the previous claims, wherein the active ingredient, the pharmaceutical formulation or the vaccine is in the form of a solid, a powder, a dust, a granulated material, capsules, microcapsules, suspension, emulsion, microparticles, pellets, crystals, minirods, nanoparticles or any other microstructure.

9. Pharmaceutical product or device according to one of the previous claims, wherein the active ingredient is selected from the group comprising chemical compounds, extracts of natural origin like plant extracts or fractions thereof and biomolecules like proteins, peptides, antigenes, antibodies, steroids, hormones, nucleic acids, DNA, RNA, oligonucleotides, attenuated virus or bacteria, vaccines, the named molecules themselves or being in a living organism, part of a living organism, a virus or part of a virus, DNA, RNA or other biotech molecules or parts of such molecules, hybrid molecular mixtures of many of such molecules, viruses, parts of viruses or bacteria.

10. Pharmaceutical product or device according to one of the previous claims, wherein the surface is a smooth surface or a structured surface.

11. Pharmaceutical product or device according to one of the previous claims, wherein the surface is a solid support or a membrane.

12. Pharmaceutical product or device according to one of the previous claims, wherein the surface is metal membrane, like an aluminium membrane or a titan membrane, a plastic material, like Polypropylene (PP), Polyethylene (PE), Polyoxymethylene (POM), a Polyether ether keton (PEEK) or Teflon.

13. Pharmaceutical product or device according to one of the previous claims for use for intra-dermal or intra-mucosal delivery, for immunisation, preferably for epidermal immunisation, like for example intra-dermal immunisation, intra-mucosal immunisation or epidermal powder immunisation (EPI).

14. Pharmaceutical product or device according to one of the previous claims comprising an acceleration device, a pyrotechnically driven powder injection device, a ballistic accelerator or a ballistic powder injection device and wherein the active ingredient, the pharmaceutical formulation or vaccine is attached to the surface of the acceleration device, the pyrotechnically driven powder injection device, the ballistic accelerator or the ballistic powder injection device by the oily substance.

15. Method of attaching an active ingredient, a pharmaceutical formulation or a vaccine to a surface of a pharmaceutical product or a device comprising the steps
a) enclosing particles of the active ingredient, the pharmaceutical formulation or the vaccine into a film of an oily substance thereby producing partly or full covered particles of the active ingredient, the pharmaceutical formulation or the vaccine and
b) bringing the partly or fully covered particles of the active ingredient, the pharmaceutical formulation or the vaccine into contact with the surface and
c) thereby attaching the active ingredient, the pharmaceutical formulation or the vaccine to the surface.

16. Method of preparing a device or a pharmaceutical product comprising the steps
a) enclosing particles of an active ingredient, a pharmaceutical formulation or a vaccine into a film of an oily substance thereby producing partly or full covered particles of the active ingredient, the pharmaceutical formulation or vaccine and
b) bringing the partly or fully covered particles of the active ingredient, the pharmaceutical formulation or the vaccine into contact with the surface of the device or the pharmaceutical product and
c) thereby attaching the active ingredient, the pharmaceutical formulation or the vaccine to the surface of the device or the pharmaceutical product.
